# EUROPÄISCHE PATENTSCHRIFT

(11) **EP 0 804 171 B1**
(45) Veröffentlichungstag und Bekanntmachung des Hinweises auf die Patenterteilung: **05.01.2000**
(21) Anmeldenummer: 96927540.3
(22) Anmeldetag: 18.07.1996
(51) Int. Cl.: A61K 9/46

(54) **KAUTABLETTE MIT BRAUSEWIRKUNG**
CHEWING TABLETS WITH AN EFFERVESCENT ACTION
COMPRIME A MACHER A ACTION EFFERVESCENTE

(30) Priorität: 31.07.1995 CH 223095
(43) Veröffentlichungstag der Anmeldung: 05.11.1997
(73) Patentinhaber: Gergely, Gerhard, Dr., A-1053 Wien (AT)
(72) Erfinder: GERGELY, Gerhard, A-1053 Wien (AT); GERGELY, Irmgard, A-1053 Wien (AT); GERGELY, Thomas, A-1053 Wien (AT)
(74) Vertreter: Büchel, Kurt F., Dr.
(86) Internationale Anmeldenummer: EP9603165
(87) Internationale Veröffentlichungsnummer: WO9704754

(56) Entgegenhaltungen:
- EP-A- 0 351 353
- EP-A- 0 415 326
- WO-A-91/04757

## Beschreibung

Die Erfindung betrifft eine Kau- oder Lutschtablette mit Brausewirkung nach dem Oberbegriff des Anspruchs 1. Solche Tabletten hat man entwickelt, um im Mund mit dem Speichel während des Auflösens ein angenehm prickelndes Gefühl zu erzeugen. Sie sind z.B. in der EP-A1-525.388 oder in der WO91/04757 beschrieben.

Die Erfinder haben sich nun jedoch die Aufgabe gestellt, eine Tablette für pharmazeutische Zubereitungen zu schaffen, die sowohl direkt oral als Kau- oder Lutschtablette mit schwachem Brause-Effekt und daher angenehm prickelndem Gefühl im Mund einzunehmen ist, als auch in (vorzugsweise nur 50-100 ml) Wasser, insbesondere in weniger als 2 min, unter Brausen aufgelöst bzw. suspendiert werden kann. Es ist nämlich im hohen Grade wünschenswert, eine beim Kauen oder Lutschen das angenehm prickelnde Gefühl erzeugende Tablette zu haben, die aber bei Bedarf, wenn der Patient doch lieber eine Lösung trinkt, unter Brausewirkung in einer (möglichst geringen) Menge Wasser gelöst werden kann. Die eingangs erwähnten Tabletten sind allerdings dazu ungeeignet, vor der Einnahme in Wasser aufgelöst zu werden: die Tablette nach der EP-A1-525.388 hat für diesen Zweck zu geringe Mengen an Brausebasis, da die Brausekomponenten schon bis zu zwei Drittel durchreagiert sind, d.h. dass von der Zitronensäure gegebenenfalls nur mehr eine einzige Carboxylgruppe frei ist; sämtliche Tabletten nach den Beispielen der WO91/04757 zeigen in frischem Wasser Auflösezeiten von weit über 5 Minuten.

Dies ist - wie die Erfinder der vorliegenden Anmeldung überraschend festgestellt haben - unter anderem auf drei Umstände in der Rezeptierung zurückzuführen, die von den Erfindern der WO91/04757 nicht beachtet worden waren, weil sie ja nur um eine Kau- oder Lutschtablette besorgt waren, die laut Beschreibung sich in Wasser von 37°C in weniger als 10 Minuten auflösen sollte. Die angegebene allfällige Untergrenze von 30 Sekunden kann mit der Rezeptierung der WO91/04757 nicht erreicht werden.

Ein erster Grund dafür ist die laut Beschreibung zwingend erforderliche Verwendung eines Gleitmittels zur Tablettierung. Ein auch relativ geringer Gehalt an Gleitmittel macht die einzelnen Bestandteile hydrophob und trägt damit ganz wesentlich zu einem schlechteren Auflöseverhalten der Tablette im Wasser bei.

Ein zweiter Grund ist die Verkapselung bzw. Beschichtung der Wirkstoffteilchen: das Coating-Material dringt beim Pressen in die Zwischenräume und blockiert dadurch auch die Brausepartikel, die dann viel langsamer mit dem Wasser reagieren können und daher die Auflösung der Tablette erschweren.

Ein dritter Grund ist die Verwendung von relativ grossen Mengen rasch löslicher Substanzen als Füllstoffe, wie z.B. Sorbit allein oder in Mischung mit einer als Presshilfsmittel rezeptierten Saccharose, die wiederum - wenn auch nur geringe Mengen - Gleitmittel enthält.

Überraschenderweise hat sich nämlich gezeigt, dass relativ rasch lösliche Füllstoffe in einer Tablette mit verhältnissmässig kleinem Gehalt an Brausebasis (wegen der allfälligen direkt oralen Einnahme der Tablette darf der Brauseeffekt nicht zu gross sein, sonst wird er als unangenehm empfunden!) die Auflösezeit der Tablette verlängern. Der Grund dafür ist, dass die im Vergleich zur Menge der Brausebasis grossen Mengen schnell löslicher Füllstoffe beim Einbringen der Tablette im Wasser sehr rasch eine lokal sehr hoch konzentrierte Lösung bilden, in der die Auflösung und Reaktion der Brausekomponenten, und damit der Zerfall der Tablette nur langsam zustande kommt.

Andererseits: lässt man das Gleitmittel und/oder die Verkapselung des Wirkstoffes beiseite, erzielt man unter Umständen auch mit geringen Mengen an Brausekomponenten, die für die Auflösung der Tablette in frischem Wasser notwendig sind, schon einen unangenehm starken Brauseeffekt im Mund, wenn die Tablette gekaut oder gelutscht werden soll.

Auch Versuche, die eingangs geschilderte Aufgabenstellung durch eine mit wenig Brausebasis dotierte Zerfallsbrause, z.B. nach EP-A1-501.985, zu lösen, schlugen fehl, da die Zerfallsmittel immer einen sandigen, rauhen Eindruck beim Kauen oder Lutschen hinterlassen, der als unangenehm empfunden wird.

Die Erfinder haben dies anhand der Beispiele nachgewiesen und die genannten Probleme durch die im Kennzeichen des Anspruchs 1 und/oder des Anspruchs 2 beschriebenen Massnahmen behoben. Vorteilhafte Weiterbildungen und Alternativen der Erfindung sind in den Kennzeichen der abhhängigen Ansprüche beschrieben.

Die geringen Wassermengen im Speichel, in dem ja noch andere Substanzen gelöst sind, lösen einen Hydrokolloid-Überzug auf den Brausekomponenten nicht so schnell weg, als dass es zu einer unerwünscht starken Brausewirkung käme. Schon geringe Mengen des Hydrokolloids bremsen somit die Reaktion der Brausekomponenten miteinander im Munde ganz drastisch, während das Hydrokolloid - insbesonders ein an sich leicht lösliches, wie z.B. Maltodextrin, Polyvinylpyrrolidon oder Guargum - sich im Wasser ganz schnell löst und die Auflösezeit einer Tablette nur unmerklich verlängert.

### Beispiel 1:

Dieses Beispiel entspricht dem Stand der Technik; das Beispiel 1 der WO91/04757 wurde nachgearbeitet, mit folgenden Variationen:
a. Acetaminophen verkapselt, mit Gleitmittel (Mg-stearat)
b. Acetaminophen verkapselt, ohne Gleitmittel
c. Acetaminophen unverkapselt, mit Gleitmittel (Mg-stearat)
d. Acetaminophen unverkapselt, ohne Gleitmittel
e. Placebo (ohne Wirkstoff), mit Gleitmittel (Mg-stearat)
f. Placebo (ohne Wirkstoff), ohne Gleitmittel

Diese sechs Systeme wurden alternativ mit vier verschiedenen Füllstoffen versetzt:
a. je 400 mg Comprizucker® und Sorbit (entsprechend genau dem Beispiel 1 der WO91/04757)
b. 800 mg Comprizucker® allein
c. 800 mg Sorbit allein
d. je 400 mg Sorbit und Mannit.

| | Acetaminophen verkapselt | | Acetaminophen unverkapselt | | Placebo (ohne Wirkstoff) | |
|---|---|---|---|---|---|---|
| | a) mit Mg-stearat | b) ohne Mg-Stearat | c) mit Mg-stearat | d) ohne Mg-stearat | e) mit Mg-stearat | f) ohne Mg-stearat |
| a) Comprizucker (400 mg) + Sorbit (400 mg) | nach 5 min ist die Tablette so gut wie nicht aufgelöst | nach 5 min ist die Tablette so gut wie nicht aufgelöst | nach 5 min ist die Tablette noch fast ganz | Auflösezeit 140 sec | nach 5 min ist die Tablette zu 3/4 gelöst | Auflösezeit 140 sec |
| b) Sorbit (800 mg) | nach 5 min ist die Tablette so gut wie nicht aufgelöst | nach 5 min ist die Tablette so gut wie nicht aufgelöst | nach 5 min ist die Tablette noch fast ganz | Auflösezeit 160 sec | nach 5 min ist die Tablette zu 3/4 gelöst | Auflösezeit 170 sec |
| c) Comprizucker (800 mg) | nach 5 min ist die Tablette noch fast ganz | nach 5 min ist die Tablette noch fast ganz | nach 5 min ist die Tablette noch fast ganz | Auflösezeit 135 sec | nach 5 min ist die Tablette zu 3/4 gelöst | Auflösezeit 100 sec |
| d) Mannit (400 mg) + Sorbit (400 mg) | nach 5 min ist die Tablette noch fast ganz | nach 5 min ist die Tablette noch fast ganz | nach 5 min ist die Tablette halb gelöst | Auflösezeit 125 sec | nach 5 min ist die Tablette fast gelöst | Auflösezeit 95 sec |

Wie aus der Tabelle ersichtlich, ist mit verkapseltem Wirkstoff und Gleitmittel die Tablette jedenfalls 5 Minuten nach dem Einbringen in 100 ml frisches Wasser so gut wie nicht aufgelöst oder nur leicht angelöst.

Selbst mit unverkapseltem Wirkstoff, aber mit Gleitmittel ist die Tablette 5 Minuten nach dem Einbringen in 100 ml frisches Wasser so gut wie nicht aufgelöst oder nur halb gelöst. Erst mit unverkapseltem Wirkstoff und ohne Gleitmittel beträgt die Auflösezeit - in Abhängigkeit vom verwendeten Füllstoff - immerhin noch 135 bis 165 sec, und nur wenn die Hälfte des Sorbits durch Mannit ersetzt wird, nur mehr 120 sec.

Aber sogar die Placebo Variante zeigt, wie störend die vorgeschlagenen Füllstoffe Comprizucker® und Sorbit sind. Zusammenfassend: sowohl die Wirkstoffverkapselung wie auch das Gleitmittel haben einen ungünstigen Einfluss auf das Auflöseverhalten; ausserdem wirkt sich schon der Ersatz des Comprizuckers® durch Mannit positiv aus, und man erzielt Auflösezeiten von immerhin 2 Minuten, was aber für die Praxis immer noch zu hoch ist.

### Beispiel 2:

Man stellt zunächst eine Brausebasis wie folgt her: 86 Teile Zitronensäure pulvis, 8,3 Teile Äpfelsäure, 95 Teile Natriumbicarbonat, 13,8 Teile Natriumcarbonat <0,1 mm, 5,5 Teile Natriumcyclamat, 1,1 Teile Saccharin-Natrium und 0,3 Teile Aspartam werden in einem auf 60°C beheizten Vakuumkessel gemischt. Daneben wird eine Lösung aus 60 Teilen Maltodextrin, 12 Teilen Trinatriumcitrat-dihydrat in 27 Teilen Wasser bereitet. Von dieser Lösung werden 4,5 Teile (entsprechend 1,3 Teilen Maltodextrin auf Brausebasis bezogen - die Umhüllung führt dazu, dass beim Lutschen die Zitronensäure nicht als so sauer empfunden wird, jedoch die Tablette sich genügend schnell auflöst) in 5 Zyklen auf die Pulvermischung im Vakuumkessel gedüst und unter schwingendem Mischen gleichmässig verteilt, wobei gleichzeitig eine Granulierung stattfindet. Nach jedem Zyklus wird im Vakuum getrocknet und anschliessend durch ein 2mm-Sieb ausgetragen. Zweckmässig werden nach dem letzten Zyklus vor der Trocknung auf das entstandene Granulat nochmals 5 Gewichtsteile Maltodextrin aufgebracht, unter Rühren gleichmässig verteilt und - vorzugsweise im Vakuum - unter langsamem Rühren getrocknet.

180 Teile der so hergestellten Brausebasis werden mit 720 Teilen Mannit, 100 Teilen Acetylsalicylsäure <0,3 mm und 0,3 Teilen Docusat-Natrium-Aerosol OTB-Pulver, sowie 0,5 Teilen Polyvinylpyrrolidon K25, 3,0 Teilen Vanillin-Aroma und 0,3 Teilen Menthol gemischt und zu Tabletten von je 1 Gramm verpresst. Die Tabletten ergeben bei direkt oraler Einnahme ein angenehmes Prickeln im Mund, bzw. lösen sich in 100 ml Wasser von 15-20°C in 55 Sekunden auf.

Das Maltodextrin kann auch durch ein anderes Hydrokolloid ersetzt werden, wie z.B. Polyvinylpyrrolidon, Guar Gum oder Dextrin (letzteres ist aber schlechter löslich), weniger gut durch Tragacanth (weil es zu stark schäumt), und bewirkt auf den Brausekomponenten eine geringfügig gebremste, "weiche" Reaktion im Mund, ohne aber - aufgrund seiner guten Löslichkeit - die Auflösung in Wasser gravierend zu verlangsamen. Diese Massnahme wirkt sich insbesondere gut bei Zitronensäure aus, wird aber zweckmässig auch bei Äpfelsäure, Weinsäure, Adipinsäure und Ascorbinsäure angewendet.

Theoretisch könnte man auch schnell lösliche Füllstoffe mit Hydrokolloid überziehen, jedoch ist dies ein technologisch aufwendiger Schritt, da das durch die Lösung des Hydrokolloids in die Mischung eingebrachte Wasser wieder weggetrocknet werden muss, und es ist die Art und Menge des Hydrokolloids hinsichtlich der Lösegeschwindigkeit schwierig zu optimieren.

Eine Mischung mit denselben Verhältnissen, in der nur die Zitronensäure zur Gänze durch Äpfelsäure ersetzt ist, ergibt völlig analoge Resultate.

### Beispiel 3:

Mit jeweils 250 mg der nach Beispiel 2 hergestellten Brausebasis und je 10 mg Nifedipin pro Tablette werden Mischungen hergestellt und zu Tabletten verpresst, wobei die Art des Füllstoffes (jeweils 750 mg) variiert wird: Alle Tabletten lösen sich in 100 ml frischem Wasser bei 16° C mit wenig bis keinem Rückstand auf und verhalten sich bei direkt oraler Einnahme als Kautablette gut bis sehr gut.

| | Füllstoff (je 750 mg) | Härte | Auflösezeit (sec) |
|---|---|---|---|
| a. | Mannit | 5,8 | 45 |
| b. | Maltisorb® (=hydriertes Maltitol) | 6,2 | 45 |
| c. | Fructose | 6,1 | 125 |
| d. | Xylit | 6,0 | 60 |
| e. | Lactose | 6,3 | 40 |
| f. | Saccharose | 5,9 | 75 |
| g. | Glukose | 5,9 | 105 |
| h. | Sorbit Instant | 5,9 | 180 |

Es ist deutlich zu sehen, dass die schnell löslichen Füllstoffe Glucose, Fructose, und erst recht Sorbit die Auflösezeit (aus den eingangs genannten Gründen) drastisch verlängern. Die verwendeten Substanzen hatten folgende Siebanalysen:

| Gew.% | Mannit | Malti sorb® | Lactose | Fructose | Sorbit | Xylit |
|---|---|---|---|---|---|---|
| Korngrösse(mm) | | | | | | |
| > 0,5 | | | | 2,5 | | |
| > 0,4 | | | | 5,5 | 0,85 | 5,4 |
| > 0,315 | | | | 4,3 | 9,0 | 33,2 |
| > 0,2 | 2,3 | 2,8 | | 29,2 | 48,1 | 60,2 |
| > 0,1 | 27,5 | 12,5 | 5,5 | 30,5 | 33,0 | 1,5 |
| > 0,05 | 36,5 | 51,6 | 29,6 | 22,4 | 8,5 | |
| > 0,05 | 33,7 | 32,7 | 64,5 | 5,6 | 1,0 | |

Die langsamer löslichen ersten drei Füllstoffe können in Feinkorn eingesetzt werden, ohne die Auflösezeit gravierend zu verlängern; die rascher löslichen letzten drei Füllstoffe müssen in gröberen Partikeln verwendet werden, wobei Sorbit wie ersichtlich mit drei Minuten Auflösezeit trotzdem noch unbefriedigend bleibt.

### Beispiel 4:

Es werden Mischungen analog zum Beispiel 3 hergestellt, mit dem Unterschied, dass - für verschiedene Wirkstoffe - die Füllstoffe in Art und Menge je nach dem Wirkstoff variiert und mit der Brausebasis auf jeweils 1000 mg pro Tablette ergänzt werden. Alle Mischungen wurden zu Tabletten mit der Härte 4,5 - 6,0 verpresst; sie lösen sich in 100 ml frischem Wasser (15-20°C) mit wenig bis keinem Rückstand auf (mit Ausnahme von gegebenenfalls schwebenden Teilchen oder einer leichten Trübung im Fall von unlöslichen oder schwerlöslichen Wirkstoffen) und verhalten sich bei direkt oraler Einnahme als Kautablette gut bis sehr gut:

| | Wirkstoff (mg) | Füllstoff(e) (mg) | Brausebasis (mg) | t_{lösl} (sec) |
|---|---|---|---|---|
| a) | 100 Acetylsalisäure (entsprechend Beispiel 2) | 700 Mannit 110 (Aroma, Süßstoffe, PVP etc.) | 190 | 55 |
| b) | 10 Nifedipin | 720 Mannit 40 (Aroma, Süßstoffe, PVP etc.) | 240 | 50 |
| c) | 100 Allopurinol | 500 Xylit 20 (Aroma, Süßstoffe, PVP etc.) | 480 | 75 |
| d) | 40 Furosemid | 720 Mannit, 10 PVP | 240 | 100 |
| e) | 10 Cisaprid | 740 Mannit 20 (Aroma, Süßstoffe, PVP etc.) | 240 | 60 |
| f) | 10 Loratadin | 90 Lactose 540 Mannit 110 (Aroma, Süßstoffe, PVP etc.) | 260 | 90 |
| g) | 45 Multivitaminmischung | 440 Xylit 390 Mannit 20 (Aroma, Süßstoffe, PVP etc.) | 150 | 60 |

### Beispiel 5:

Aus geschmacklichen Gründen ist oft eine Mischung verschiedener Füllstoffe erwünscht. Das Beispiel 3 wurde wiederholt, wobei verschiedene Füllstoff-Mischungen zum Einsatz kamen. Solche Mischungen erlauben unter Umständen auch den Einsatz schneller löslicher Füllstoffe wenigstens zu einem Teil:

| | Füllstoff | Härte | Auflösezeit (sec) |
|---|---|---|---|
| a. | Mannit (500mg)/ Sorbit (250mg) | 4,0 | 70 |
| b. | Mannit (500mg)/ Fructose (250mg) | 4,0 | 75 |
| c. | Xylit (375mg)/ Lactose (375mg) | 4,5 | 45 |
| d. | Xylit (525mg)/ Lactose (225mg) | 4,0 | 45 |
| e. | Fructose (375mg)/ Lactose (375mg) | 4,5 | 55 |
| f. | Fructose (600mg)/ Lactose (150mg) | 4,5 | 95 |
| g. | Glucose (375mg)/ Lactose (375mg) | 5,5 | 70 |
| h. | Glucose (600mg)/ Lactose (150mg) | 4,5 | 95 |

### Beispiel 6:

Die Mischung des Beispiels 4 9) wird wiederholt, mit dem Unterschied, dass als Füllstoff nur Mannit, dieser aber in wechselnden Mengen bzw. Verhältnissen zur Brausebasis eingesetzt wird. Es zeigt sich, dass sowohl die Auflösezeit in Wasser wie auch die Wirkung als Kautablette im Mund bei Verhältnissen von Brausebasis : Mannit = 15:85 bis 30:70 als gut bis sehr gut einzustufen ist. Bei einem Verhältnis von 35-40 : 65-60 hingegen liegt zwar die Auflösezeit natürlich erfreulich niedrig; jedoch wird die Brausewirkung im Mund bereits als zu stark bzw. der Geschmack als zu sauer empfunden:

| Brausebasis | Füllstoff | Kautablette | Härte | Auflösezeit (sec) |
|---|---|---|---|---|
| | | | | |

| (Gewichtsteile) | | | | |
|---|---|---|---|---|
| 15 | 85 Mannit | sehr gut | 5,0 | 65 |
| 20 | 80 Mannit | sehr gut | 5,5 | 55 |
| 25 | 75 Mannit | gut | 5,5 | 45 |
| 30 | 70 Mannit | gut | 5,0 | 40 |
| 35 | 65 Mannit | anfangs sauer | 5,5 | 35 |
| 40 | 60 Mannit | zu sauer | 5,0 | 35 |

### Beispiel 7:

Die Mischungen des Beispiels 5 g) und h) wurden, mit einer noch weiteren Variation der Mischungsverhältnisse zwischen Glucose und Lactose einerseits, der Härte der verpressten Tabletten andererseits wiederholt; es ergibt sich wie ersichtlich auch bei grösserer Tablettenhärte eine noch akzeptable Auflösezeit; auch diese Tabletten waren bei direkt oraler Einnahme gut bis sehr gut zu kauen bzw. zu lutschen:

| Füllstoff (mg) | Härte (kp) | Auflösezeit (s) | Rückstand |
|---|---|---|---|
| Glucose 750 | 5,9 | 105 | keiner |
| Glucose 562 | 5,9 | 70 | keiner |
| Lactose 188 | | | |
| Glucose 375 | 6,3 | 70 | Spur |
| Lactose 375 | | | |
| Glucose 188 | 6,4 | 65 | Spur |
| Lactose 562 | | | |
| Lactose 750 | 6,3 | 40 | etwas |

### Beispiel 8 (Negativ-Beispiel):

Sorbit allein ist so rasch löslich, insbesondere auch im zwar grobkörnigen, aber infolge der Sprühtrocknung porösen Sorbit Instant, dass - wie bereits erwähnt - mit Sorbit allein als Füllstoff produzierte Tabletten eine unerwünscht lange Auflösezeit ergeben, und zwar unabhängig von der Korngrösse:

| Füllstoff (mg) | Härte (kp) | Auflösezeit (s) | Rückstand |
|---|---|---|---|
| Sorbit Instant | 6,5 | 150 | keiner |
| Sorbit P300 | 6,5 | 150 | keiner |
| Sorbit 60W (kristallin) | 6,5 | 160 | keiner |

### Beispiel 9:

In einen aufheizbaren Kessel werden 468 g Zitronensäure pulvis, 312 g Zitronensäure Feingries und 150 g Natriumcyclamat eingebracht und unter Rühren auf 60°C aufgeheizt. Bei 60°C erfolgt die Zugabe von 420 g Natriumbicarbonat. Die Masse wird unter Rühren wieder auf 60°C aufgeheizt.

Daneben stellt man 90 ml einer Lösung aus 60 g Maltodextrin, 12 g Trinatriumcitrat-dihydrat und 59 g Wasser her. Man saugt nun 9 ml dieser Lösung ein und lässt anreagieren; anschliessend gibt man bei 100 mbar 19 g Natriumcarbonat zu und trocknet unter langsamem Rühren auf 20 mbar. Nach dem Trocknen wird wieder auf 60°C aufgeheizt, und es erfolgt eine zweite Granulierung mit 9 ml der Lösung. Die Masse wird unter langsamem Rühren auf 100 mbar getrocknet, worauf weitere 77 g Natriumcarbonat zugefügt werden. Anschliessend wird das Produkt auf 15 mbar getrocknet und auf 1,6 mm gesiebt. Diese Brausebasis wird hierauf mit 2784 g Mannit, 600 g Xylit, 1200 g Lactose, 120 g Aroma und 135 g Isosorbid-5-Mononitrat (90%ige Lactose-Verreibung) vermischt und zu Tabletten von je 1,05 g verpresst.

### Beispiel 10:

Man geht analog Beispiel 2 vor, nur verwendet man statt Zitronensäure Weinsäure und statt Acetylsalicylsäure eine Standard-Multivitamin-Mischung, wie z.B. 50% der RDA (=Recommended Daily Allowance) nach US Standard, und zwar von Vitamin E Acetat (Tocopherolacetat), Thiaminmononitrat, Pyridoxin-HCl, Riboflavinphosphat-Natrium, Nicotinamid, Folsäure, Biotin, Calciumpantothenat, Vitamin D3 100CWS, Vitamin B12 0.1%ig und Vitamin A Palmitat CWS, sodass die fertige Tablette folgende Zusammensetzung (in mg) aufweist:

| | |
|---|---|
| Weinsäure Pulver | 86,00 |
| Äpfelsäure | 8,30 |
| Natriumbicarbonat | 95,00 |
| Natriumcarbonat wasserfrei | 13,80 |
| Natriumcyclamat | 5,50 |
| Saccharin-Natrium | 1,10 |
| Aspartam | 0,30 |
| Trinatriumcitrat-dihydrat | 0,95 |
| Maltodextrin | 1,00 |
| Mannit | 700,00 |
| Multivitamin-Mischung | 45,50 |
| Aroma | 20,00 |
| | 977,45 |

Die Tablette zeigt im Mund ein befriedigendes Verhalten und löst sich in einem Glas Wasser in weniger als 60 sec auf.

Die Erfindung lässt sich bei vielen Wirkstoffen anwenden, deren Dosierung bis ca. 200 mg betragen kann. Besonders vorteilhaft sind Wirkstoffe, deren Dosierung nur bis zu 100 mg beträgt, da eine kleine Tablette zum Lutschen oder Kauen besser geeignet ist als eine grössere.

## Patentansprüche

1. Kautablette mit Brausewirkung und einem Tablettengewicht unter 3 g, enthaltend wenigstens einen pharmazeutischen Wirkstoff, 15 bis 50 Gewichtsprozent einer Brausebasis aus einer festen, essbaren, organischen Säure und einem Alkali- und/oder Erdalkalikarbonat und/oder -bikarbonat, sowie 30 bis 85 Gewichtsprozent eines löslichen Füllstoffs, dadurch gekennzeichnet, dass die Säure- und/oder die Carbonat-Teilchen der Brausebasis mit einem - vorzugsweise leicht bzw. schnell löslichen - Hydrokolloid überzogen sind.

2. Kautablette mit Brausewirkung und einem Tablettengewicht unter 3 g, enthaltend wenigstens einen pharmazeutischen Wirkstoff, 15 bis 50 Gewichtsprozent einer Brausebasis aus einer festen, essbaren, organischen Säure und einem Alkali- und/oder Erdalkalikarbonat und/oder -bikarbonat, sowie 30 bis 85 Gewichtsprozent eines löslichen Füllstoffs, dadurch gekennzeichnet, dass der Wirkstoff frei von Verkapselungssubstanzen ist und/oder die Tablettenmischung frei von Gleitmitteln ist.

3. Kautablette nach einem der vorhergehenden Ansprüche, dadurch gekennzeichnet, dass die Brausekomponenten mit 0,5 bis 3,0, vorzugsweise von 1 bis 2 Gewichtsprozent des Hydrokolloids, insbesondere mit Maltodextrin, Polyvinylpyrrolidon und/oder Guargum, überzogen sind.

4. Kautablette nach Anspruch 1 oder 2, dadurch gekennzeichnet, dass der Füllstoff wenigstens eine der folgenden Verbindungen enthält: Mannit, hydriertes Maltitol, Fructose, Xylit, Lactose, Saccharose, Glucose.

5. Kautablette nach einem der vorhergehenden Ansprüche, dadurch gekennzeichnet, dass die Füllstoffkombination so abgestimmt ist, dass die Tablette - mit Ausnahme allenfalls unlöslicher oder schwerlöslicher Wirkstoffe - sich in 100 ml Wasser von 15 bis 20°C in weniger als 120, vorzugsweise höchstens 90 Sekunden auflöst.

6. Kautablette nach einem der vorhergehenden Ansprüche, dadurch gekennzeichnet, dass 40 bis 100 Gewichtsprozent rascher löslicher Füllstoffe, wie Fructose, Xylit oder Sorbit, eine Korngrösse über 0,2 mm aufweisen, während 60 bis 95 Gewichtsprozent langsamer löslicher Füllstoffe, wie Mannit, hydrierte Maltose oder Lactose, eine Korngrösse unter 0,1 mm aufweisen.

7. Kautablette nach einem der vorhergehenden Ansprüche, dadurch gekennzeichnet, dass das Tablettengewicht 600 bis 1500 mg, vorzugsweise 700 bis 1200 mg, beträgt.

8. Kautablette nach einem der vorhergehenden Ansprüche, dadurch gekennzeichnet, dass die Menge der Brausebasis in einer Tablette 100 bis 600, vorzugsweise 150 bis 350 mg beträgt.

9. Kautablette nach einem der vorhergehenden Ansprüche, dadurch gekennzeichnet, dass höchstens die Hälfte der Füllstoffmenge durch Sorbit ersetzt ist.

10. Kautablette nach einem der vorhergehenden Ansprüche, dadurch gekennzeichnet, dass ein Teil, vorzugsweise etwa 5 bis etwa 20 Gewichtsprozent, der Säure durch eine zweite, von ihr verschiedene, feste, essbare, organische Säure, vorzugsweise Äpfelsäure, ersetzt ist.

11. Kautablette nach einem der vorhergehenden Ansprüche, dadurch gekennzeichnet, dass sie 55 bis 75 Gew.% wenigstens eines Füllstoffes, 15 bis 25 Gew.% einer Brausebasis und 15 bis 25 Gew.% von Isosorbid-5-Mononitrat und/oder Isosorbid-5-Dinitrat enthält

12. Verfahren zur Herstellung einer Kautablette nach einem der Ansprüche 1 oder 3 bis 11, , dadurch gekennzeichnet, dass die Brausekomponenten bei erhöhter Temperatur mit einer wenigstens ein Hydrokolloid enthaltenden Lösung benetzt und wieder getrocknet werden, welcher Zyklus vorzugsweise ein- bis fünfmal wiederholt wird, worauf die so hergestellte Brausebasis mit Wirk- und/oder Hilfsstoffen gemischt und zu Tabletten verpresst wird.

13. Verfahren nach Anspruch 12, dadurch gekennzeichnet, dass vor der Trocknung nach dem letzten Zyklus 0.2 bis 1, vorzugsweise 0.4 bis 0.6 Gewichtsprozent Hydrokolloid in Pulverform aufgebracht werden.

## Claims

1. Chewable tablet with an effervescent action and a tablet weight of less than 3 g, containing at least one pharmaceutical active substance, 15 to 50 percent by weight of an effervescent base comprising a solid, edible, organic acid and an alkali metal and/or alkaline earth metal carbonate and/or bicarbonate and 30 to 85 percent by weight of a soluble filler, characterized in that the acid particles and/or the carbonate particles of the effervescent base have been coated with a - preferably readily or rapidly soluble - hydrocolloid.

2. Chewable tablet with an effervescent action and a tablet weight of less than 3 g, containing at least one pharmaceutical active substance, 15 to 50 percent by weight of an effervescent base comprising a solid, edible, organic acid and an alkali metal and/or alkaline earth metal carbonate and/or bicarbonate and 30 to 85 percent by weight of a soluble filler, characterized in that the active substance is free of encapsulating substances and/or the tablet mixture is free of lubricants.

3. Chewable tablet according to any of the preceding Claims, characterized in that the effervescent components are coated with 0.5 to 3.0, preferably 1 to 2, percent by weight of the hydrocolloid, in particular with maltodextrin, polyvinylpyrrolidone and/or guar gum.

4. Chewable tablet according to Claim 1 or 2, characterized in that the filler contains at least one of the following compounds, mannitol, hydrogenated maltitol, fructose, xylitol, lactose, sucrose and glucose.

5. Chewable tablet according to any of the preceding Claims, characterized in that the filler combination is tailored so that the tablet - with the exception of any insoluble or sparingly soluble active substances - dissolves in 100 ml of water at 15 to 20°C in less than 120, preferably not more than 90, seconds.

6. Chewable tablet according to any of the preceding Claims, characterized in that in that 40 to 100 percent by weight of more rapidly soluble fillers, such as fructose, xylitol or sorbitol, have a particle size of more than 0.2 mm while 60 to 95 percent by weight of more slowly soluble fillers, such as mannitol, hydrogenated maltose or lactose, have a particle size of less than 0.1 mm.

7. Chewable tablet according to any of the preceding Claims, characterized in that the tablet weight is 600 to 1500 mg, preferably 700 to 1200 mg.

8. Chewable tablet according to any of the preceding Claims, characterized in that the amount of the effervescent base in a tablet is 100 to 600, preferably 150 to 350, mg.

9. Chewable tablet according to any of the preceding Claims, characterized in that not more than half of the amount of filler is replaced by sorbitol.

10. Chewable tablet according to any of the preceding Claims, characterized in that a part, preferably about 5 to about 20 percent by weight, of the acid is replaced by a second, solid, edible, organic acid differing from said acid, preferably malic acid.

11. Chewable tablet according to any of the preceding Claims, characterized in that it contains 55 to 75 percent by weight of at least one filler, 15 to 25% by weight of an effervescent base and 15 to 25% by weight of isosorbide 5-mononitrate and/or isosorbide 5-dinitrate.

12. Process for the preparation of a chewable tablet according to any of Claims 1 or 3 to 11, characterized in that the effervescent components are wet at elevated temperature with a solution containing at least one hydrocolloid and are dried again, which cycle is preferably repeated once to five times, after which the effervescent base thus prepared is mixed with active substances and/or excipients and compressed to give tablets.

13. Process according to Claim 12, characterized in that 0.2 to 1, preferably 0.4 to 0.6, percent of hydrocolloid in powder form is applied before the drying after the last cycle.

## Revendications

1. Comprimé à mâcher à action effervescente avec un poids de comprimé inférieur 3 grammes, contenant au moins un agent pharmaceutique actif, 15 à 50 pour-cent en poids d'une base effervescente constituée par des acides organiques alimentaires solides et un carbonate et/ou un bicarbonate de métal alcalin et/ou alcalino-terreux, ainsi que de 30 à 85 pour-cent en poids d'un excipient soluble, caractérisé en ce que la portion acide et/ou carbonate de la base effervescente est recouverte, de préférence, d'un hydrocolloïde peu soluble ou rapidement soluble.

2. Comprimé à mâcher à action effervescente avec un poids de comprimé inférieur à 3 g, contenant au moins un agent pharmaceutique actif, 15 à 50 pour-cent en poids d'une base effervescente constituée par des acides organiques alimentaires solides et un carbonate et/ou un bicarbonate de métal alcalin et/ou alcalino-terreux, ainsi que de 30 à 85 pour-cent en poids d'un excipient soluble, caractérisé en ce que l'agent actif est exempt de substance d'encapsulation et/ou que le mélange pour comprimé est exempt d'agent lubrifiant.

3. Comprimé à mâcher selon l'une des revendications précédentes, caractérisé en ce que les composants effervescents sont recouverts par 0,5 à 3,0 et, de préférence, 1 à 2 pour-cent en poids d'un hydrocolloïde, en particulier par de la maltodextrine, de la polyvinylpyrrolidone et/ou de la gomme de guar.

4. Comprimé à mâcher selon la revendication 1 ou la revendication 2, caractérisé en ce que l'excipient contient au moins un des composés suivants : mannitol, maltose hydraté, fructose, xylitol, lactose, saccharose, glucose.

5. Comprimé à mâcher selon l'une des revendications précédentes, caractérisé en ce que la combinaison des excipients est choisie de manière à ce que le comprimé - exception faite toutefois d'un agent actif insoluble ou peu soluble - se dissolve dans 100 ml d'eau à 15 - 20 °C en moins de 120 secondes et, de préférence, au maximum en 90 secondes.

6. Comprimé à mâcher selon l'une des revendications précédentes, caractérisé en ce que de 40 à 100 pour-cent en poids de l'excipient à dissolution rapide, tel que le fructose, le xylitol ou le sorbitol, a une granulométrie supérieure à 0,2 mm, alors que 60 à 95 pour-cent en poids de l'excipient à dissolution plus lente tel que le mannitol, le maltose hydraté ou le lactose, a une granulométrie inférieure à 0,1 mm.

7. Comprimé à mâcher selon l'une des revendications précédentes, caractérisé en ce que le poids d'un comprimé est de 600 à 1500 mg et, de préférence, de 700 à 1200 mg.

8. Comprimé à mâcher selon l'une des revendications précédentes, caractérisé en ce que la quantité de la base effervescente d'un comprimé est de 100 à 600 et, de préférence, de 150 à 350 mg.

9. Comprimé à mâcher selon l'une des revendications précédentes, caractérisé en ce qu'au maximum la moitié de l'excipient est constituée par du sorbitol

10. Comprimé à mâcher selon l'une des revendications précédentes, caractérisé en ce qu'une partie et, de préférence, d'environ 5 à environ 20 pour-cent en poids de l'acide est remplacé par un second acide organique alimentaire solide différent du premier, qui est, de préférence, l'acide malique.

11. Comprimé à mâcher selon l'une des revendications précédentes, caractérisé en ce qu'il contient de 55 à 75 % en poids d'au moins un excipient, de 15 à 25 % d'une base effervescente et de 15 à 25 % en poids de 5-mononitrate d'isosorbide et/ou de 5-dinitrate d'isosorbide.

12. Procédé pour fabriquer un comprimé à mâcher selon la revendication 1 ou une des revendications 3 à 11, caractérisé en ce que les composants effervescents sont mouillés à une température augmentée avec une solution contenant au moins un hydrocolloïde et ensuite séchés, ce cycle étant répété de préférence d'une à cinq fois, suite à quoi la base effervescente ainsi préparée est mélangée avec des substances actives et/ou des adjuvants, et le mélange pressé en comprimés.

13. Procédé selon la revendication 12, caractérisé en ce qu'avant le séchage du dernier cycle, on ajoute de 0,2 à 1 et, de préférence, de 0,4 à 0,6 pour-cent en poids d'un hydrocolloïde sous une forme pulvérulente.
